# FASCICULE DE BREVET EUROPEEN

(11) **EP 3 917 554 B1**
(45) Date de publication et mention de la délivrance du brevet: **19.07.2023**
(21) Numéro de dépôt: 20701215.4
(22) Date de dépôt: 27.01.2020
(51) Int. Cl.: A61K 36/53, A61K 8/9789, A61K 9/00, A61K 8/34, A61K 8/36, A61K 8/49, A61K 8/63, A61Q 19/08, A61P 17/16

(54) **PROCEDE D'OBTENTION D'UN EXTRAIT DE FEUILLES DE PATCHOULI ET SES UTILISATIONS COSMETIQUES**
VERFAHREN ZUR HERSTELLUNG EINES EXTRAKTS AUS PATCHOULI-BLÄTTERN UND KOSMETISCHE VERWENDUNGEN DAVON
METHOD FOR OBTAINING AN EXTRACT OF PATCHOULI LEAVES AND COSMETIC USES THEREOF

(30) Priorité: 29.01.2019 FR 1900813
(43) Date de publication de la demande: 08.12.2021
(73) Titulaire: ISP Investments LLC, Wilmington, Delaware 19805 (US); Jafer Enterprises R&D, S.L.U., 08403 Granollers (Barcelona) (ES)
(72) Inventeur: IMBERT, Isabelle, 06400 CANNES (FR); COQUET, Corinne, 06620 CIPIERES (FR); GONDRAN, Catherine, 03330 BELLENAVES (FR); LABARRADE, Florian, 06600 ANTIBES (FR); BORSOTTO, Jérémie, 06580 PEGOMAS (FR); GARNIER, Sébastien, 06740 Chateauneuf Grasse (FR); DUROURE, Leslie, 06370 MOUANS SARTOUX (FR)
(74) Mandataire: Miquel, Corinne
(86) Numéro de dépôt international: PCT/EP2020/051883
(87) Numéro de publication internationale: WO 2020/156981

(56) Documents cités:
- WO-A1-2018/167038
- US-A1- 2014 271 525
- US-B1- 6 387 416
- LIN RONG-FENG ET AL: "Prevention of UV radiation-induced cutaneous photoaging in mice by topical administration of patchouli oil", JOURNAL OF ETHNOPHARMACOLOGY, ELSEVIER IRELAND LTD, IE, vol. 154, no. 2, 18 avril 2014 (2014-04-18), pages 408-418, XP029028509, ISSN: 0378-8741, DOI: 10.1016/J.JEP.2014.04.020 cité dans la demande
- DONELIAN A ET AL: "Comparison of extraction of patchouli (Pogostemon cablin) essential oil with supercritical CO"2 and by steam distillation", THE JOURNAL OF SUPERCRITICAL FLUIDS, ELSEVIER, AMSTERDAM, NL, vol. 48, no. 1, 1 février 2009 (2009-02-01), pages 15-20, XP025924407, ISSN: 0896-8446, DOI: 10.1016/J.SUPFLU.2008.09.020 [extrait le 2009-01-29]
- GONDRAN C ET AL: "Evaluation of an extract of Pogostemon cablin (patchouli) on the cannabinoid receptor CB2 and associated reduction of skin discomfort sensation", JOURNAL OF INVESTIGATIVE DERMATOLOGY, vol. 139, no. 9, Suppl. S, septembre 2019 (2019-09), page S260, XP055650519, & 49TH ANNUAL MEETING OF THE EUROPEAN-SOCIETY-FOR-DERMATOLOGICAL-RESEAR CH (ESDR); BORDEAUX, FRANCE; SEPTEMBER 18 -21, 2019

## Description

### Domaine technique

La présente invention concerne le domaine de la cosmétique et plus particulièrement celui des ingrédients actifs entrant dans la formulation des compositions pour les soins de la peau. L'invention a trait à un procédé d'obtention d'un extrait particulier de feuilles de patchouli (*Pogostemon cablin*). L'invention concerne également des extraits de feuilles de patchouli comprenant de 50 à 80 % de composés volatils, de 2 à 5% de composés phénoliques et de 15 à 48% de composés lipidiques obtenus par un tel procédé, les compositions cosmétiques comprenant de tels extraits, et enfin l'utilisation cosmétiques de telles compositions pour le soin de la peau, du cuir chevelu et des phanères.

### Arrière-plan technique de l'invention

Le patchouli (*Pogostemon cablin*) est une plante tropicale de la famille des Lamiacées principalement cultivée en Asie et en particulier en Indonésie, d'environ 1 mètre de haut, aux feuilles larges et veloutées. Après séchage, le patchouli possède une odeur puissante, à la fois boisée et terreuse avec des accents fumés et camphrés.

Le patchouli est surtout utilisé en parfumerie et en cosmétique, sous forme d'huile essentielle produite par simple distillation des feuilles ou par des techniques d'extraction plus complexes. Il est par exemple connu du document KR1034010B1 un procédé d'obtention de dérivés d'huile essentielle de patchouli par distillation reproduite 2 à 4 fois puis ajout d'hexane et enfin concentration par chromatographie. On connaît également une méthode d'extraction d'huile de patchouli comprenant une étape de traitement microbien de feuilles et de tiges sèches de patchouli (brevet US7879584B2) .

Les composés majoritaires des huiles essentielles de patchouli sont le patchoulol, gamma-guaiène, alpha-guaiène, alpha-patchoulène and béta-caryophyllène. Une huile essentielle obtenue par extraction à la vapeur d'eau contient de 30 % à 40 % de patchoulol parmi des dizaines d'autres composés (Donelian A. et al. Comparison of extraction of patchouli (Pogostemon cablin) essential oilwith supercritical CO2 and by steam distillation; J. Supercritical Fluids 48 (2009) 15-20).

L'huile essentielle de patchouli comprend également, selon les études de Yahya et Yunus (Yahya, A and Yunus R, Influence of sample préparation and extraction time on chemical composition of steam distillation derived patchouli oil. Procedia Engineering, 2013; 53: 1-6) des sesquiterpènes : alpha-bulnésène (20-25%), alpha-guaiène (10-12%), beta-patchoulène (2-3%), béta-caryophyllène (3-4%) et des alcools sesquiterpéniques comme le pogostol (2-3%).

Le patchouli est connu et utilisé en médecine ayurvédique pour le réconfort de l'esprit et la reconnexion avec son moi profond (Swamy MK et al. A Comprehensive Review on the Phytochemical Constituents and Pharmacological Activities of Pogostemon cablin Benth.: An Aromatic Medicinal Plant of Industrial Importance. Molecules. 2015 May 12; 20(5):8521-47) . En occident, il est associé au mouvement hippie des années 1970.

En aromathérapie, le patchouli est utilisé pour détendre et soulager les dépressions (Ramya H. G. et al. An introduction to patchouli (Pogostemon cablin Benth.) -A medicinal and aromatic plant: It's importance to mankind. July, 2013 Agric Eng Int: CIGR Journal Open access at http://www.cigrjournal.org Vol. 15, No.2 243) et également comme tonique, stimulant digestif et tonique circulatoire.

Le patchouli est également connu pour être anti-inflammatoire, antiémétiques, antalgiques et antiseptiques et pour ses effets sur la peau et les cheveux. On connait par exemple une composition pour favoriser la croissance des cheveux préparée par macération hydroalcoolique des feuilles sèches de plante *Pogostemonis, Hélianthes annus* et *Cinnamoni cortex,* citée dans le document de *brevet* KR2008081393A, ou encore une composition nettoyante à base de patchouli obtenue par extraction supercritique et du polyglucosides d'origine naturelle, décrit dans le document WO2010086717.

L'huile essentielle de patchouli est également connue pour prévenir le photo-vieillissement grâce à ses propriétés antioxidantes (Lin RF. Prévention of UV radiation-induced cutaneous photoaging in mice by topical administration of patchouli oil ; J Ethnopharmacol. 2014 Jun 11;154(2):408-18).

La peau est un organe composé de plusieurs couches (derme, épiderme et *stratum corneum*), qui recouvre toute la surface du corps et assure des fonctions protectrices vis-à-vis des agressions extérieures, sensitives, immunitaires, métaboliques, thermorégulatrices ou encore de barrière limitant la déshydratation.

L'aspect de la peau peut être modifié par des altérations internes (vieillissement intrinsèque, maladies et changements hormonaux tels que la grossesse) ou externes (facteurs environnementaux, tels que la pollution, la lumière du soleil, les pathogènes, les variations de température, etc.). Toutes ces altérations affectent non seulement la peau, mais aussi les annexes kératiniques telles que les poils, les cils, les sourcils, les ongles et les cheveux.

Le système cannabinoïde endogène ou endocannabinoïde tire son nom de de la plante qui a permis sa découverte ; le cannabis. Ce système est présent chez tous les vertébrés, dans de nombreux organes où II participe à la régulation d'un nombre très vaste de processus physiologiques, dont le développement neural, l'inflammation, l'immunité, l'appétit, le métabolisme, la perception des informations sensorielles, notamment nociceptives, les cycles de veille et de sommeil ainsi que la régulation du stress et de l'état émotionnel.

La peau et ses annexes (follicule pileux, glande sébacée) sont dotés de leur propre système cannabinoïde complet et fonctionnel, comprenant les ligands endocannabinoïdes, leurs récepteurs CB1 et CB2 associés aux protéines G, ainsi que leurs enzymes de synthèse et de métabolisation ((Ashton JC et al. ; Tamàs Bíró,Balázs I. Tóth,1 Gyôrgy Haskó, Ralf Paus and Pàl Pocher. The endocannabinoid system of the skin in health and disease: novel perspectives and therapeutic opportunities. Trends Pharmacol Sci. 2009 Aug; 30(8): 411-420). Le système endocannabinoïde de la peau intervient en particulier dans les fonctions de régulation de la prolifération et de la différenciation des cellules de l'épiderme, et la modulation de l'inflammation. En complément, les agonistes des récepteurs CB2 stimulent la synthèse de la beta-endorphine, connue pour son effet anti-douleur (Su et al. Cannabinoid CB2 Receptors Contribute to Upregulation of b-endorphin in Inflamed Skin Tissues by Electroacupuncture. Molecular Pain 2011, 7:98. Ibrahim M. M. CB2 cannabinoid receptor activation produces antinociception by stimulating peripheral release of endogenous opioids. Proc Natl Acad Sci USA. 2005, February 22, vol.102, no.8, 3093-3098).

De nouvelles molécules agissant sur les récepteurs CB2, donc non liées à l'effet psychotrope ont été récemment développées (Klein TW. Cannabinoid-based drugs as anti-inflammatory therapeutics. Nature reviews. 2005 May ;5, 400-411). Les cannabinoïdes exogènes peuvent aussi être utilisés par voie topique pour leur propriété anti-inflammatoire (Mounessa JS, Siegel JA, Dunnick CA, Dellavalle RP. The rôle of cannabinoids in dermatology. J Am Acad Dermatol. 2017 Jul;77(1):188-190). Cette activité anti-inflammatoire a également été rapportée pour le THC (Δ9-tetrahydrocannabinol), composant principal de *Cannabis sativa.* Le THC peut exercer un effet anti-inflammatoire et anti-douleur via les récepteurs cannabinoïdes CB1 et CB2. L'effet psychotrope du THC est quant à lui médié par les récepteurs cannabinoïdes CB1.

Dans le domaine de la cosmétique le développement de nouveaux extraits de plantes pouvant agir sur le système endocannabinoïde de la peau et avoir des effets apaisants présente également un très grand intérêt.

Les inventeurs ont ainsi mis en évidence qu'un nouvel extrait particulier de patchouli, obtenu par un procédé d'extraction permettant à la fois d'extraire les composés volatils, les composés phénoliques et les composés lipidiques, présentait des effets apaisants et protecteurs sur la peau et les cheveux. L'extrait de l'invention est différent d'une huile essentielle de patchouli classique par son contenu en composés phénoliques, représentés par un mélange de flavonoïdes non glycosylés, et en composés lipidiques, représentés principalement par un mélange d'acides gras, de phytostérols, de triterpènes et d'acyls glycérides. Or il a été démontré que cette composition particulière de l'extrait renforce son activité biologique par rapport à une huile essentielle de patchouli classique.

### Résumé de l'invention

L'invention a pour premier objet un procédé d'obtention d'un extrait de feuilles séchées de patchouli comprenant les étapes suivantes :
a) La partie haute des parties aériennes de patchouli est récoltée puis séchée et broyée ou cryobroyée.
b) On réalise une extraction au dioxyde de carbone supercritique en présence d'éthanol à une concentration comprise entre 60 et 80% (pourcentage volume/volume), en tant que co-solvant polaire.
c) On récupère l'extrait de patchouli qui est décoloré en présence de charbon actif et filtré au préalable de l'évaporation du co-solvant,
d) L'extrait brut obtenu à l'étape c) est solubilisé dans un solvant de type alcool gras saturé ou insaturé, linéaire ou ramifié, comprenant de 8 à 30 carbones pour obtenir une concentration d'extrait brut dans l'extrait final comprise entre 2 et 6 % en poids du poids total de l'extrait solubilisé.

L'invention a pour deuxième objet un extrait brut de patchouli susceptible d'être obtenu par le procédé selon l'invention comprenant entre 50 et 80% de composés volatils principalement de type sesquiterpènes et alcools sesquiterpéniques, entre 2 et 5% de composés phénoliques de type flavonoïdes non glycosylés, et entre 15 et 48% de composés lipidiques comme notamment les acides gras et les phytostérols.

L'invention a également pour objet un extrait solubilisé de patchouli comprenant de 2 à 6% d'extrait brut solubilisé dans un solvant de type alcool gras saturé ou insaturé, linéaire ou ramifié, comprenant de 8 à 30 carbones

L'invention a pour troisième objet une composition cosmétique pour le soin de la peau, du cuir chevelu et des phanères, comprenant comme agent actif, un extrait de patchouli obtenu selon le procédé de l'invention et un milieu physiologiquement acceptable.

L'invention a pour quatrième objet l'utilisation cosmétique d'une composition comprenant l'extrait de patchouli de l'invention pour améliorer l'aspect de la peau, lutter contre les signes du vieillissement cutané ou pour améliorer l'hydratation de la peau et renforcer la fonction barrière ou encore apaiser la peau.

### Brève description des dessins

L'invention et les avantages qui en découlent seront mieux compris à la lecture de la description et des modes de réalisation non limitatifs qui suivent, illustrés au regard des dessins annexés dans lesquels :

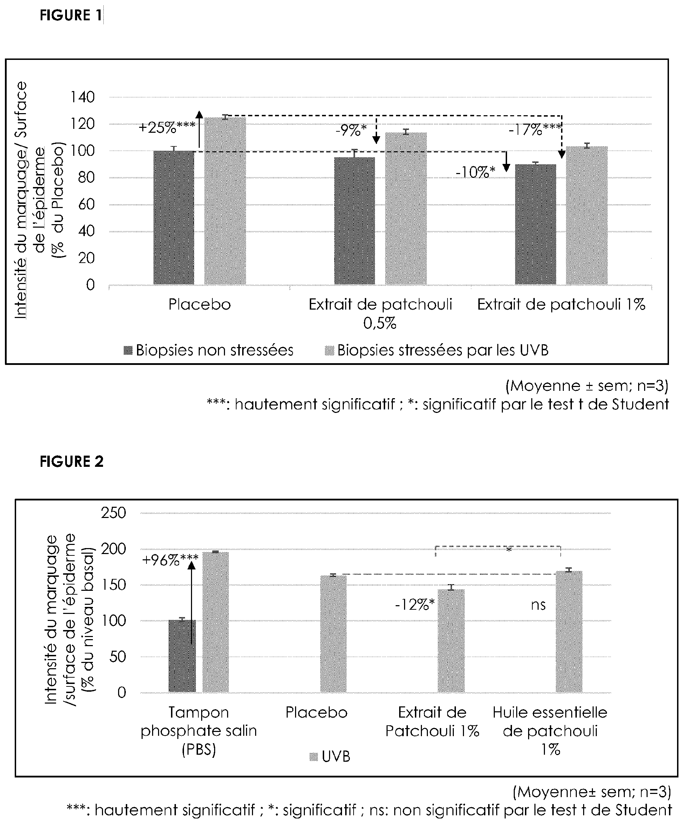

**Figure 1** - Quantification du marquage de I'IL1-R1 (récepteur de l'interleukine 1) sur biopsies de peau exposées aux UVB et traitées par l'extrait de patchouli
   La figure 1 représente l'effet de l'extrait de patchouli sur le taux d'IL1-R1 évalué sur des biospies de peau, après une irradiation par les UVB
**Figure 2** - Quantification du marquage de I'IL1-R1 (récepteur de l'interleukine 1) sur biopsies de peau exposées aux UVB et traitées par l'extrait de patchouli ou par l'huile essentielle de patchouli
   La figure 2 représente les résultats obtenus avec l'extrait de patchouli et une huile essentielle de patchouli sur le taux d'IL1-R1 évalué sur des biopsies de peau, après une irradiation par les UVB

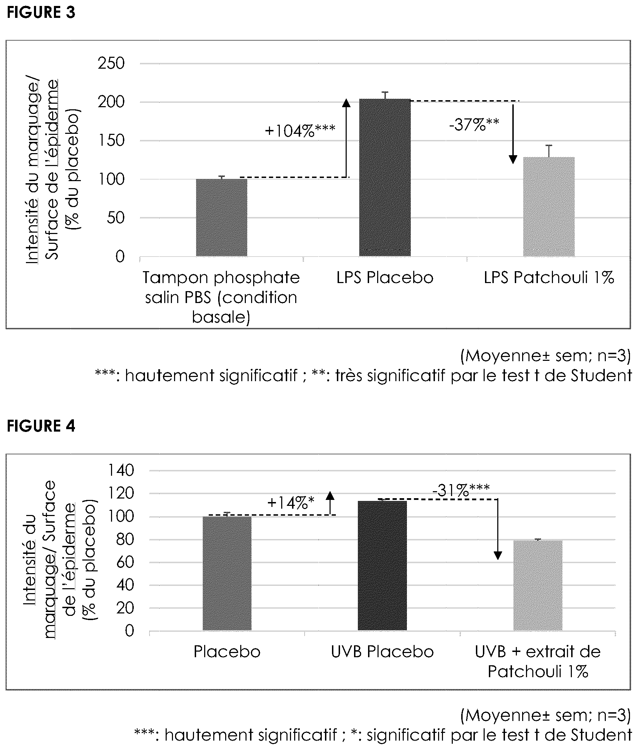

**Figure 3** - Quantification du marquage de I'IL1-R1 (récepteur de l'interleukine 1) sur biopsies de peau exposées au lipopolysaccharide bactérien et traitées par l'extrait de patchouli
   La figure 3 représente les résultats obtenus avec l'extrait de patchouli sur le taux d'IL1-R1 évalué sur des biopsies de peau exposées au lipopolysaccharide d'origine bactérienne
**Figure 4** - Quantification du marquage de TRPV1 sur biopsies de peau exposées aux UVB et traitées par l'extrait de patchouli
   La figure 4 représente les résultats obtenus avec l'extrait de patchouli sur le récepteur TRPV1, associé à la réponse aux cannabinoïdes, sur des biopsies de peau exposées à une irradiation par les UVB

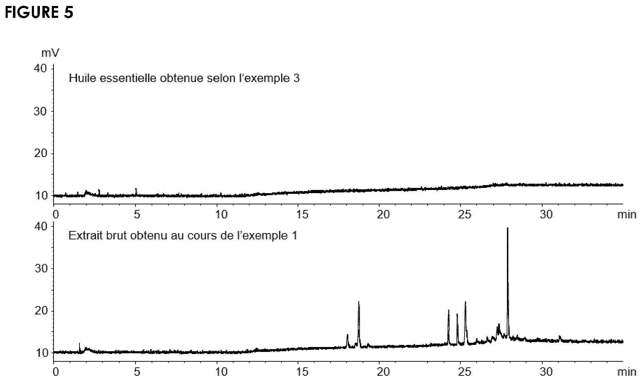

**Figure 5** - Profils chromatographiques d'une huile essentielle de patchouli obtenue selon l'exemple 3 et de l'extrait brut de patchouli de l'exemple 1, en CLHP/DEDL sur une colonne RP-C18 avec élution en mode gradient (0-8 min 100% A, 8-25 min passage de 100% A à 100% B puis 25-35 min 100% B - A : H2O/ACN/HCO2H 95/5/0.1 (v:v:v) et B : IPA/ACN/HCO2H 80/20/0.1 (v:v:v)). On retrouve sur l'axe des ordonnée la réponse du détecteur en mV et en abscisse le temps d'analyse en minutes.

### Description détaillée de l'invention

### Procédé d'extraction

Des méthodes d'extractions du patchouli utilisant le CO₂ supercritique sont décrites dans plusieurs documents. Le document CN101485647B (également publié sous le numéro WO2010096987) décrit un extrait de patchouli extrait par l'alcool ou le CO₂ supercritique et de nouveaux usages pharmaceutiques. Le Patchouli est réduit en poudre et soumis à une extraction par du CO₂ supercritique dans les conditions suivantes pression 12-30 Mpa ; température 55-60 °C. Ces extraits de patchouli contiennent de l'alcool de patchouli (patchoulol).

On connaît également le document IN200900195I1 (également publié sous le numéro WO2010086717) qui décrit une composition nettoyante à base de plantes comprenant des extraits de plantes certifiées bio obtenus par extraction supercritique et du polyglucosides d'origine naturelle.

Donelian A. et collègues (J. Supercritical Fluids 48 (2009) 15-20) décrivent une extraction par CO₂ supercritique des feuilles de Patchouli séchées et broyées dans les conditions suivantes : températures 40 et 50_{°}C, pressions 8.5 et 14 MPa, et flux de CO2 6.0×10-3 kg/min, pendant une période de 340 min. Les analyses chromatographiques montrent que les conditions d'extraction à 14MPa à 40_{°}C, produisent l'huile essentielle la plus concentrée en patchoulol et le plus haut rendement d'extraction.

On connaît du travail de Hybertson B. M. (J Chem Eng Data. 2007 January 1; 52(1)) que le taux de patchoulol extrait par CO₂ supercritique varie en fonction de la température et de la pression appliquées. Les conditions donnant la plus forte concentration de patchoulol sont 40_{°}C, et 25 MPa. Un fluide est dit supercritique lorsqu'il est placé dans des conditions de température et de pression au-delà de son point critique (Tc, Pc). Les propriétés physiques d'un fluide supercritique (densité, viscosité, diffusion, diffusivité) sont intermédiaires entre celles des liquides et celles des gaz, mais leurs propriétés de dissolution sont considérablement accrues. Il est bien connu d'utiliser du dioxyde de carbone à l'état supercritique car il présente l'avantage d'être un solvant totalement neutre, non toxique, non inflammable, pouvant être mis en oeuvre à une température assez basse (31°C) pour une pression supérieure à sa pression critique de 73,8 bar. Cette technique permet de travailler à une température modérée (à partir de 31°C), ce qui ne dénature pas les qualités organoleptiques et les principes actifs de l'extrait obtenu. De plus, après évaporation du CO₂ revenu à l'état gazeux, elle permet d'obtenir des extraits exempts de tous résidus du solvant d'extraction. Il est aussi possible d'utiliser d'autres fluides dont notamment l'argon.

Néanmoins aucun document de l'état de l'art ne décrit la méthode d'extraction de l'invention utilisant comme solvant à la fois du dioxyde de carbone à l'état supercritique et une solution d'éthanol/eau.

Le procédé d'extraction objet de l'invention utilise la partie haute des parties aériennes séchée de patchouli comprenant les étapes suivantes :
a) La partie haute des parties aériennes de patchouli est récoltée puis séchée et broyée ou cryobroyée.
b) On réalise une extraction au dioxyde de carbone supercritique en présence d'éthanol à une concentration comprise entre 60 et 80% (pourcentage volume/volume), en tant que co-solvant polaire.
c) On récupère l'extrait de patchouli qui est décoloré en présence de charbon actif et filtré au préalable de l'évaporation du co-solvant,
d) L'extrait brut obtenu est solubilisé dans un solvant de type alcool gras saturé ou insaturé, linéaire ou ramifié, comprenant de 8 à 30 carbones.
e) Éventuellement on purifie l'extrait obtenu à l'étape c) ou d).

La partie haute des parties aériennes de patchouli est récoltée, puis les feuilles et les tiges les plus fines sont séchées et broyées ou cryobroyées.

Le terme « partie haute des parties aériennes » est défini comme les feuilles et les tiges les plus fines, après avoir écarté le matériel végétal en mauvais état et les tiges trop grosses.

Au sens de la présente description, « partie haute des parties aériennes » ne comprend pas les fruits, les fleurs et les graines.

Dans le cours de la présente description, les termes « Les parties hautes des parties aériennes » et « feuilles » seront utilisés indifféremment pour désigner les feuilles et les tiges les plus fines.

Préférentiellement, l'extrait est obtenu à partir de plante *Pogostemon cabiin* cultivée en Colombie.

Pour réaliser l'étape a) la partie haute des parties aériennes de patchouli est récoltée puis séchée. De préférence, les feuilles et les tiges sont cryobroyées dans un broyeur à couteaux comportant une grille de 4 mm, ce qui permet d'obtenir une poudre dont la taille des particules se situe entre 100 et 800 µm, avantageusement entre 300 et 600 µm et plus préférentiellement entre 400 et 500 µm.

Pour réaliser l'étape b) le fluide à l'état supercritique est choisi parmi le dioxyde de carbone.

Selon un mode de réalisation préférentiel, de l'étape b) :
- Le co-solvant polaire est l'éthanol à une concentration de 70% (70 % volume/volume dans l'eau).
- La température d'extraction à l'étape b) est comprise entre 35 et 70°C, avantageusement entre 40 et 65°C et préférentiellement entre 50 et 60°C.
- La pression au sein de l'extracteur est comprise entre 90 et 1000 bar, préférentiellement entre 160 et 800 bar et encore plus préférentiellement entre 230 et 700 bar.
- Le rapport massique de solvant supercritique (dioxyde de carbone) par rapport à la quantité de matière première mise en jeu (feuilles séchées et broyées de patchouli) est compris entre 10 et 70, avantageusement entre 25 et 55 et préférentiellement entre 30 et 50.

Pour réaliser l'étape b) avantageusement, la poudre obtenue à l'étape a) est placée dans une cartouche en acier inoxydable, cette cartouche est placée dans un extracteur à fluide supercritique. Le solvant utilisé pour extraire est le dioxyde de carbone à l'état supercritique. Dans le même temps, un co-solvant tel que l'éthanol est également injecté dans la cartouche d'extraction. Le ratio massique entre le dioxyde de carbone et le co-solvant est de 15.

A l'étape c) l'extrait obtenu à l'étape b) est décoloré en présence de charbon actif, puis filtré. Le co-solvant éthanol/eau est évaporé. Le rendement d'extraction est ainsi proche de 6%.

A ce stade l'extrait brut de feuilles de patchouli est sous forme pâteuse et comprend entre 50 et 80% de composés volatils, entre 2 et 5% de composés phénoliques et entre 15 et 48% de composés lipidiques.

A l'étape d) l'extrait brut est solubilisé dans un solvant agro-sourcé tel qu'un alcool gras saturé ou insaturé, linéaire ou ramifié, comprenant de 8 à 30 carbones.

De préférence le solvant de type l'alcool gras est l'octyldodécanol capable de solubiliser toutes les familles de composés décrits dans l'extrait. Après cette étape de solubilisation, l'extrait se présente sous la forme d'une solution limpide et fluide contenant entre 2 et 6% d'extrait brut de feuilles de Patchouli.

De préférence l'extrait solubilisé contient 4% d'extrait brut de feuilles de Patchouli.

L'octyldodécanol, sert de solvant et de support liquide cosmétiquement acceptable aux composés d'intérêt. Par agro-sourcé, on entend des molécules provenant totalement ou partiellement de la biomasse, en effet ces solvants sont composés de carbone renouvelable.

Optionnellement une étape e) de purification de l'extrait obtenu à l'étape c) peut être effectuée par toute technique connue de l'homme du métier et notamment par chromatographie ou par distillation moléculaire

### Extrait

L'extrait susceptible d'être obtenu par le procédé après solubilisation à l'étape d) est sous forme liquide et il contient un mélange de molécules d'intérêt possédant des polarités très variées. L'analyse de l'extrait montre que les molécules extraites s'avèrent différentes en qualité et en quantité de celles décrites dans l'état de l'art. L'extrait de l'invention est différent d'une huile essentielle de patchouli classique par son contenu en composés phénoliques, comme les flavonoïdes, et en composés lipidiques, comme les phytostérols et les acides gras. Or il a été démontré dans l'exemple 7 de la présente demande que cet extrait présentait une efficacité supérieure pour apaiser la peau qu'une huile essentielle de patchouli classique.

Ainsi, le deuxième objet de l'invention est un extrait de patchouli susceptible d'être obtenu par le procédé selon l'invention.

On entend par « extrait brut » de patchouli l'extrait sous forme pâteuse obtenu à l'étape b) du procédé

On entend par « extrait » de patchouli ou « extrait solubilisé » au sens de l'invention l'extrait liquide obtenu après solubilisation à l'étape c) du procédé.

L'extrait ainsi obtenu selon l'invention est une solution limpide jaune pâle de consistance huileuse.

L'extrait de patchouli est composé notamment de :
- de 2 à 6% d'extrait brut en poids du poids total de l'extrait solubilisé
- Préférentiellement, de 4% d'extrait brut, en poids du poids total de l'extrait de patchouli.

L'extrait brut comprend lui-même :
- de 50 à 80% de composés volatils (principalement des sesquiterpènes et des alcools sesquiterpéniques), avantageusement de 55 à 75% et préférentiellement de 60 à 70%
- de 2 à 5% de composés phénoliques (principalement des flavonoïdes non glycosylés), avantageusement 2.5 à 4.5% et préférentiellement de 3 à 4%
- de 15 à 48% de composés lipidiques (notamment des acides gras, phytostérols, triterpènes, acyl glycérides), avantageusement 20.5 à 42.5% et préférentiellement de 26 à 32%
- Dans un mode de réalisation très avantageux, l'extrait brut comprend 65 % de composés volatils, 3,5 % de composés phénoliques et 31,5% de composés lipidiques.
- Par « composés volatils » on entend des molécules suffisamment légères pour être détectées par chromatographie en phase gazeuse. Il s'agit principalement de sesquiterpènes et d'alcools sesquiterpéniques de formule brute respective C₁₅H₂₄ et C₁₅H₂₆O présentes dans l'extrait
- Par « composés phénoliques » on entend des flavonoïdes non glycosylés détectés dans l'extrait.
- Par « composés lipidiques » on entend le groupe des molécules de nature apolaires appartenant aux familles des acides gras, des triterpènes, des stérols et des acyls glycérides présentes dans l'extrait.

Chaque fraction de l'extrait brut a été analysée afin de déterminer les principales molécules pouvant posséder une activité biologique sur la peau.

Les composés volatils sont analysés par chromatographie en phase gazeuse (GC) couplé à un détecteur de spectrométrie de masse (MS) et/ou à un détecteur à ionisation de flamme (FID). La validation des identifications est rendue possible par comparaison des indices de rétention linéaire et des spectres de masse contenus dans des librairies. La quantification par GC/FID est faite par étalonnage interne avec utilisation des facteurs de réponse prédits et calculés.

Les composés phénoliques et lipidiques sont suivis par injection en chromatographie liquide à haute performance (CLHP) couplé à un détecteur évaporatif à diffusion de lumière (DEDL). Les identifications sont confirmées après analyse de résonnance magnétique nucléaire (RMN) et/ou MS des composés isolés avec confirmation par injection de standards quand cela est possible. Les flavonoïdes sont dosés avec un détecteur à barrette de diode (DAD/UV) par étalonnage interne avec de l'acide gallique. Après validation de la conformité du profil lipidique, la teneur globale en lipides (acides gras, stérols, triterpènes, acyls glycérides...) est estimée par soustraction des teneurs en volatils et en flavonoïdes.

Les molécules majoritairement identifiées sont :
Dans la fraction volatile, des composés de type sesquiterpènes et alcool sesquiterpéniques comme I' -guaiène, I' -bulnesène, et le patchoulol.
Dans la fraction phénolique, des composés de type flavonoïdes non glycosylés comme le pachypodol.
Dans la fraction lipidique, des composés de type acides gras (tel que les acides linolénique, linoléique, et palmitique), phytostérols (tel que le β-sitostérol et le stigmastérol, ...), triterpènes (tel que l'acide oléanolique) et acyl glycérides.

La liste non exhaustive des composés présents dans chacune des fractions est donnée dans le tableau 1 suivant :

| Famille chimique | Identification |
|---|---|
| Composés volatils (notamment sesquiterpènes et alcools sesquiterpéniques) - Fraction A | Alpha bulnesène |
| | Patchoulol |
| | Pogostol |
| | Alpha guaiène |
| | Béta patchoulène |
| | Béta caryophyllène |
| | Seychellène |
| | Alpha patchoulène |
| | Norpatchoulenol |
| Composés phénoliques (notamment flavonoïdes) - Fraction B | Pachypodol |
| | 5-hydroxy-7,3',4'-trimethoxyflavanone |
| | Rhamnetine |
| | Rhamnazine |
| | Retusine |
| | 4',5-dihydroxy-3',7-dimethoxyflavanone |
| | 3,5-dihydroxy-4',7-dimethoxyflavone |
| | 5-hydroxy-3,4',7-trimethoxyflavone |
| Composés lipidiques (notamment acides gras, stérols, triterpènes, acyls glycérides) - Fraction C | Acide linolénique |
| | Acide oléanolique |
| | Acide linoléique |
| | Linoléate de méthyl |
| | Acide palmitique |
| | Acide oléique |
| | Acide stéarique |
| | Beta sitosterol |
| | Stigmasterol |
| | Acyl glycérides |

Quelques marqueurs de l'extrait brut ont pu être quantifiés. Celui-ci contient notamment :
- 16.1 à 25.7 % de patchoulol co-élué avec le pogostol, avantageusement 17.7 à 24.1 %, préférentiellement 19.3 à 22.5 % (Fraction A).
- 10.2 à 16.2 % d' -bulnesene, avantageusement 11.2 à 15.2 %, préférentiellement 12.2 à 14.2 % (Fraction A).
- 7.0 à 11.0 % d' -guaiene, avantageusement 7.6 à 10.4 %, préférentiellement 8.3 à 9.7 % (Fraction A).
- 1 à 2.6% de pachypodol co-élué avec la 5-hydroxy-7,3',4'-trimethoxyflavanone, avantageusement 1.3 à 2.3 % et préférentiellement 1.55 à 2.05% (Fraction B).

### Compositions cosmétiques

L'invention a pour troisième objet une composition comprenant, en tant qu'agent actif apaisant et anti-âge, une quantité efficace d'un extrait de patchouli selon l'invention, et un milieu physiologiquement acceptable.

Dans cette description, à moins qu'il n'en soit spécifié autrement, il est entendu que, lorsqu'un intervalle est donné, il inclut les bornes supérieure et inférieure dudit intervalle.

Dans cette description, à moins qu'il n'en soit spécifié autrement, les pourcentages sont donnés en poids.

Un autre objet de l'invention concerne une composition comprenant, en tant qu'agent actif anti-âge, une quantité efficace d'un extrait de patchouli susceptible d'être obtenu par le procédé selon l'invention, et un milieu physiologiquement acceptable.

Un milieu physiologiquement acceptable désigne un véhicule adapté pour une mise en contact avec les couches externes de la peau, du cuir chevelu ou des phanères, sans toxicité, irritation, réponse allergique indue et similaire ou réaction d'intolérance, et proportionné à un rapport avantage/risque raisonnable.

A titre de milieu physiologiquement acceptable communément utilisé dans le domaine d'application envisagé, on peut citer par exemple des adjuvants nécessaires à la formulation, tels que des solvants, des épaississants, des diluants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, etc.

Préférentiellement, la composition selon l'invention comprend l'extrait de patchouli susceptible d'être obtenu par le procédé selon l'invention à une concentration de 0,1 à 10% en poids par rapport au poids total de la composition, préférentiellement de 0,5% à 5%, et un milieu physiologiquement acceptable.

La composition utilisable selon l'invention pourra être appliquée par toute voie appropriée, notamment topique externe, et la formulation des compositions sera adaptée par l'homme du métier.

Préférentiellement, les compositions selon l'invention se présentent sous une forme adaptée à l'application par voie topique.

Par application topique, on désigne le fait d'appliquer ou d'étaler une composition comprenant l'extrait de patchouli de l'invention à la surface de la peau, du cuir chevelu d'une muqueuse ou des phanères.

La « peau » désigne la peau du visage, notamment le contour des yeux et la bouche, le nez, le front, le cou, les mains, mais aussi la peau de l'ensemble du corps, y compris le cuir chevelu.

Les compositions selon l'invention sont particulièrement adaptées à une application topique sur des peaux saines. Dans le cadre de la présente invention, on entend par peau saine une peau qui ne présente pas de pathologie cutanée.

Les phanères désignent les annexes cutanées kératinisées présentes chez l'homme ou l'animal, riches en kératine, et plus particulièrement les cheveux, les poils, les cils, les sourcils et les ongles.

Les compositions topiques pour la mise en oeuvre de l'invention pourront notamment se présenter sous forme d'une solution aqueuse, hydro-alcoolique ou huileuse, d'une émulsion huile-dans-eau, eau-dans-huile, émulsion multiple, micro-émulsion, nano-émulsion ou tout système colloïdal utilisable en cosmétique ; elles peuvent aussi se présenter sous forme de suspensions, ou encore de poudres, adaptées à une application sur la peau, les muqueuses, les lèvres et/ou les cheveux.

Ces compositions peuvent être plus ou moins fluides et avoir également l'aspect d'une crème, d'une lotion, d'un lait, d'un sérum, d'une pommade, d'un gel, d'une pâte ou d'une mousse. Elles peuvent aussi se présenter sous forme solide, comme un stick ou encore formulée pour être compatible avec une délivrance sous forme d'aérosol.

Dans tous les cas, l'homme de métier veillera à ce que les adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20% du poids total de la composition. Lorsque la composition selon l'invention est une émulsion, la phase grasse peut représenter de 5 à 80% en poids et de préférence de 5 à 50% en poids par rapport au poids total de la composition. Les émulsionnants et co-émulsionnants utilisés dans la composition sont choisis parmi ceux classiquement utilisés dans le domaine considéré. Par exemple, ils peuvent être utilisés en une proportion allant de 0,3 à 30% en poids par rapport au poids total de la composition.

Dans un mode de réalisation particulier les compositions peuvent contenir un ou des agents actifs additionnels pour renforcer l'effet de l'extrait de patchouli selon l'invention.

L'INCI Dictionary & Handbook ("International Nomenclature of Cosmetic Ingredients 13ème Ed. 2010) publié par "the Personal Care Products Council, Inc.", Washington, D.C.) décrit une grande variété, sans limitation, d'ingrédients cosmétiques habituellement utilisés dans l'industrie du soin de la peau, qui conviennent pour être utilisés comme agents actifs additionnels dans les compositions selon la présente invention.

Des exemples non limitatifs de ces classes d'agents actifs additionnels incluent : les agents anti-âge, les agents anti-rides, les agents hydratants, les agents adoucissants, les agents kératolytiques ou desquamants, les agents anti-séborrhéiques, les agents anti-pelliculaires, les agents modulant la différenciation ou la prolifération des cellules cutanées, les agents modulant la pigmentation cutanée, les agents auto bronzants, les agents anti-pollution atmosphérique, les agents anti-glycation, les agents raffermissants, les agents stimulant la synthèse de d'aquaporine, les agents stimulant la synthèse de lipides et de composants du stratum corneum (céramides, acides gras, les agents stimulant la prolifération des adipocytes, les agents stimulant la synthèse des glycosaminoglycanes, les agents de réparation de l'ADN, les agents protégeant l'ADN, les agents pour le traitement et/ou le soin de la peau sensible, les agents raffermissants, les agents anti-vergetures, les agents astringents, les agents dermo-relaxants, les facteurs de croissance de cytokine, les agents agissant sur la circulation et/ ou microcirculation capillaire, les agents inhibant la perméabilité vasculaire, les agents agissant sur le métabolisme cellulaire, les agents destinés à améliorer la jonction dermo-épidermique, les agents induisant la pousse des cheveux et/ou des poils, les agents stimulant la lipolyse, les agents amincissants, les agents anti-cellulite, les écrans solaires, les agents capables de réduire ou traiter les poches sous les yeux, et leurs mélanges, tant qu'ils sont physiquement et chimiquement compatibles avec les autres ingrédients de la composition et spécialement avec les actifs de la présente invention.

Par ailleurs, la nature de ces agents actifs additionnels ne doit pas altérer de manière inacceptable les bénéfices des actifs de l'invention. Ces agents actifs additionnels peuvent être synthétiques ou naturels comme par exemple les extraits de plantes ou venir d'un procédé de biofermentation.

De tels agent actifs additionnels peuvent également être choisis selon leur composition chimique, dans le groupe comprenant : les sucres aminés, glucosamine, D-glucosamine, N-acetyl- glucosamine, N-acetyl-D-glucosamine, mannosamine, N-acetyl mannosamine, galactosamine, N-acétyl galactosamine, vitamine B3 et ses dérivés, le niacinamide, déhydro-acétate de sodium, l'acide déhydroacétique et ses sels, phytosterols, composés d'acide salicylique, hexamidines, composés dihydroxyproline de dialkanoyl, extraits et dérivés de soja, equol, isoflavones, flavonoïdes, phytantriol, farnesol, géraniol, bisabolol, peptides et leurs dérivés, di -, tri -, tétra -, penta -, et hexapeptides et leurs dérivés, lys-thr-thr-lys-ser, palmitoyl-lys-thr-thr-lys-ser, carnosine, composés d'acide aminé N-acyl, rétinoïdes, propionate de rétinyl, rétinol, palmitate de rétinyl, acétate de rétinyl, rétinal, acide rétinoïque, vitamines hydrosolubles, ascorbates, vitamine C, glucoside ascorbyl, palmitate ascorbyl, magnésium ascorbyl phosphate, sodium ascorbyl phosphate, vitamines et leurs sels et dérivés, provitamines et leurs sels et dérivés, ethyl panthenol, vitamine A et ses dérivés, vitamine B et ses dérivés, vitamine BI, vitamine B2, vitamine B6, vitamine B12, vitamine E, vitamine F, vitamine K et ses dérivés, acide pantothénique et ses dérivés, éther éthylique de pantothenyl, panthenol et ses dérivés, panthenol ethylique, dexpanthenol, biotine, acides aminés et leurs sels et les dérivés, acides aminés hydrosolubles, asparagine, alanine, indol, acide glutamique, vitamines insolubles dans l'eau, bêta-ionol, cedrol, et leurs dérivés, acides aminés insolubles dans l'eau, tyrosine, tryptamine, matériaux particulaires, hydroxytoluène butylé, hydroxyanisole butylé, allantoine, nicotinate de tocophérol, tocophérol, esters de tocophérol, palmitoyl-gly-his-lys, phytostérol, hydroxy acides, acide glycolique, acide lactique, acide lactobionique, kétoacides, acide pyruvique, acide phytique, acide lysophosphatidique, stilbenes, cinnamates, resveratrol, kinétine, zeatin, dimethylaminoethanol, peptides naturels, les peptides de soja, sels de sucres acides, gluconate de manganèse, gluconate de zinc, olamine de piroctone, 3,4,4'-trichlorocarbanilide, triclocarban, pyrithione de zinc, hydroquinone, acide kojique, acide ascorbique, magnésium ascorbyl phosphate, ascorbyl glucoside, pyridoxine, l'aloe vera, les alcools de terpène, allantoine, bisabolol, glycyrrhizinate dipotassique, acide de glycérol, de sorbitol, de pentaerythritol, de pyrrolidone et ses sels, dihydroxyacetone, erythrulose, glycéraldéhyde, tartaraldehyde, l'essence de clou de girofle, le menthol, le camphre, l'essence d'eucalyptus, eugénol, le lactate de menthyle, le distillat d'hamamélis, copolymère d'eicosene et vinyl pyrrolidone, iodopropyl butylcarbamate, un polysaccharide, un acide gras essentiel, un salicylate, un acide glycyrrhétinique, des caroténoïdes, des céramides et des pseudo-céramides, un lipide complexe, les huiles en général d'origine naturelle tels le beurre de karité, l'huile d'abricot, l'huile d'onagre, l'huile de pruneau, l'huile de palme, l'huile de monoï, l'huile de kahai, hydroquinone, 1'HEPES, la procystéine, l'O-octanoyl-6-D-maltose, le sel disodique d'acide méthyl glycine diacétique, les stéroïdes tels que la diosgénine et les dérivés de la DHEA, la DHEA déhydroépiandrostérone et/ou un précurseur ou dérivé chimique ou biologique, le N-éthylcarbonyl-4-para-aminophénol, les alpha hydroxy acides, les béta hydroxy acides, les hydratants, les enzymes hydrolytiques épidermiques, les extraits de plantes, les phytohormones, les extraits de levure, un inhibiteur de métalloprotéinases, les enzymes, les inhibiteurs enzymatiques, les inducteurs enzymatiques, les coenzymes, les agents chélatants, les extraits végétaux et dérivés de plantes, les huiles essentielles, les extraits marins, les agents venant d'un procédé de biofermentation et/ou de biotechnologie, les sels minéraux, les extraits cellulaires.

A titre d'exemples, on peut encore citer :
- les peptides commercialement connus sous le nom MATRIXYL^{®}, ARGIRELINE^{®}, CHRONOGEN^{™}, LAMINIXYL IS^{™}, PEPTIDE Q10^{™}, COLLAXYL ^{™} (brevet FR2827170, ASHLAND^{®}), PEPTIDE VINCI 01^{™} (brevet FR2837098, ASHLAND^{®}), PEPTIDE VINCI 02^{™} (brevet FR2841781, ASHLAND^{®}), ATPeptide^{™} (brevet FR2846883, ASHLAND^{®}) ou encore le peptide de synthèse de séquence Arg-Gly-Ser-NH2, commercialisé sous le nom ATPeptide^{™} par ASHLAND^{®} ;
- l'extrait d'Artémia salina, commercialisé sous le nom GP4G^{™} (FR2817748, ASHLAND^{®}) ;
- les extraits peptidiques végétaux tels que les extraits de lin (Lipigénine^{™}, brevet FR2956818, ASHLAND^{®}), les extraits de soja, d'épeautre, de vigne, de colza, de lin, de riz, de maïs, de pois, de cacao ;
- les extraits de levures, par exemple le Dynagen^{™}, (brevet FR2951946, ASHLAND^{®}) ou l'Actopontine^{™} (brevet FR2944526, ASHLAND^{®}).

### Utilisations

Un dernier objet de l'invention concerne l'utilisation et les compositions selon la présente invention sont particulièrement destinées aux soins de la peau saine, du cuir chevelu et des phanères.

L'invention concerne plus particulièrement l'utilisation cosmétique de la composition selon l'invention pour améliorer l'aspect de la peau, lutter contre les signes du vieillissement cutané ou pour améliorer l'hydratation de la peau et renforcer la fonction barrière.

L'invention concerne encore l'utilisation cosmétique d'une composition selon l'invention pour apaiser la peau.

L'invention concerne également l'utilisation cosmétique d'une composition selon l'invention pour augmenter l'expression des récepteurs endocannabinoïdes CB2 dans la peau.

L'invention concerne également l'utilisation cosmétique d'une composition selon l'invention pour diminuer l'expression des récepteurs endocannabinoïdes TRPV1 dans la peau, après irradiation par les UVB.

On entend par « apaiser la peau » diminuer la sensation d'inconfort, telle que les possibles picotements, démangeaisons, sensation de chaleur représentant les désagréments liés à la peau sensible et à la peau sèche, lorsque que la peau est néanmoins considérée comme saine. On entend également par apaiser la peau diminuer les signes visibles de la peau sensible tels que la rougeur ou les squames dus à la sécheresse de la peau, ainsi que les autres signes perceptibles de la peau sensible tels qu'une peau moins lisse et moins douce au toucher.

On entend par « améliorer l'aspect de la peau » que le grain de la peau apparait plus fin, plus régulier.

Par« signes du vieillissement cutané » on entend les modifications de l'aspect extérieur de la peau dues au vieillissement comme les rides et ridules, les crevasses, les poches sous les yeux, les cernes, le flétrissement, la perte d'élasticité, de fermeté et/ou de tonus de la peau, l'irrégularité du grain de peau ou du teint, mais également toutes modifications internes de la peau qui ne se traduisent pas systématiquement par un aspect extérieur modifié comme, par exemple, l'amincissement de la peau, ou toutes dégradations internes de la peau consécutives à des stress environnementaux tels que la pollution et les rayonnements UV.

On entend par « améliorer la fonction barrière » que les propriétés de protection de la peau vis-à-vis des agression extérieures (radiations UV, pollution, microorganismes, etc.) est améliorée.

On entend par « amélioration de l'hydratation cutanée », toutes améliorations des modifications de l'aspect extérieur de la peau dues à la déshydratation comme, par exemple, la sécheresse, les tiraillements et l'inconfort.

### Exemples

La présente invention va maintenant être illustrée au moyen des exemples suivants :

### Exemple 1 : Préparation d'un extrait de patchouli selon l'invention

Pour la réalisation des exemples, les plantes *Pogostemon cablin* ont été cultivées en Colombie.

Permis : Les demandeurs se sont renseignés sur la nécessité d'obtenir un permis de Recherche et de Commercialisation auprès du gouvernement Colombien pour avoir accès aux ressources génétiques de *Pogostemon Cablin* qui a répondu qu'aucun permis n'était nécessaire.

Les feuilles sont récoltées, séchées puis cryobroyées dans un broyeur à couteaux comportant une grille de 4 mm, ce qui permet d'obtenir une poudre dont la taille des particules se situe entre 400 et 500 µm. La poudre obtenue est placée dans une cartouche en acier inoxydable, cette cartouche est placée dans un extracteur à fluide supercritique. Le solvant utilisé pour extraire est le dioxyde de carbone à l'état supercritique et de l'éthanol à 70% (70 % dans l'eau volume/volume) en tant que co-solvant polaire. Le ratio dioxyde de carbone/éthanol est de 15. La pression au sein de l'extracteur est de 400 bar. La température d'extraction est de 50°C. L'extrait ainsi obtenu est décoloré à l'aide de charbon actif puis filtré et l'éthanol est ensuite évaporé. Le rendement d'extraction est de 6%.

L'extrait pâteux, objet de la présente invention, est solubilisé dans de l'octyldodecanol agro- sourcé afin d'obtenir une solution limpide et fluide contenant entre 2 et 6% d'extrait brut de feuilles de Patchouli.

L'extrait solubilisé obtenu également objet de la présente invention, est une solution liquide, translucide et de couleur jaune pâle. Son odeur est caractéristique et le profil obtenu par CPG montre bien la présence des composés volatils de *Pogostemon cablin.* Le profil CLHP/DEDL, quant à lui, atteste bien de la présence des composés phénoliques et lipidiques caractéristiques de notre extrait. La teneur globale en composés phénoliques, déterminée par CLHP via un étalonnage interne avec de l'acide gallique, est de 0.14%.

### Exemple 2 : Caractérisation de l'extrait de patchouli brut obtenu selon l'exemple 1

L'extrait brut est engagé dans deux étapes successives de distillation moléculaire pour séparer les composés volatils, retrouvés dans le distillat, des composés phénoliques et lipidiques, retrouvés dans le résidu. Le distillat est conservé pour l'étude des composés volatils par CPG. Il constitue la fraction A.

Le résidu est ensuite déposé sur une colonne de silice, de taille adaptée à la masse d'extrait à purifier. La séparation des composés d'intérêt est faite par chromatographie flash grâce à un gradient de solvants allant de 100% heptane à 100% acétate d'éthyle. Une phase de rinçage avec du méthanol termine l'élution. Le contenu des tubes de récolte contenant des composés de même famille sont regroupés puis les solvants sont éliminés à l'évaporateur rotatif. Ce fractionnement permet d'obtenir une fraction riche fraction (fraction B) dissociée de la fraction lipidique (fraction C). Chacune de ces fractions est ensuite engagée dans un fractionnement plus affiné par CLHP préparative sur colonne C18 afin d'isoler indépendamment chaque composé. L'étude conjointe des spectres RMN et/ou et des données de spectrométrie de masse permet l'identification de plusieurs composés. Ces dernières sont confirmées par l'injection d'un standard lorsque cela est possible. La liste non exhaustive des composés présents dans chacune des fractions est présentée dans le tableau 2 suivant :

| Famille chimique | Identification |
|---|---|
| Composés volatils (notamment sesquiterpènes et alcools sesquiterpéniques) - Fraction A | Béta patchoulène |
| | Béta caryophyllène |
| | Seychellène |
| | Alpha guaiène |
| | Alpha patchoulène |
| | Alpha bulnesène |
| | Norpatchoulenol |
| | Patchoulol |
| | Pogostol |
| Composés phénoliques (notamment flavonoïdes) - Fraction B | Rhamnetine |
| | Rhamnazine |
| | Pachypodol |
| | Retusine |
| | 4',5-dihydroxy-3',7-dimethoxyflavanone |
| | 3,5-dihydroxy-4',7-dimethoxyflavone |
| | 5-hydroxy-7,3',4'-trimethoxyflavanone |
| | 5-hydroxy-3,4',7-trimethoxyflavone |
| Composés lipidiques (notamment acides gras, stérols, | Acide linolénique |
| | Acide oléanolique |
| triterpènes, acyls glycérides) - Fraction C | Acide linoléique |
| | Linoléate de méthyl |
| | Acide palmitique |
| | Acide oléique |
| | Acide stéarique |
| | Beta sitosterol |
| | Stigmasterol |
| | Acyl glycérides |

Quelques marqueurs de l'extrait brut ont pu être quantifiés. Celui-ci contient notamment :
- 20.9 % de patchoulol co-élué au pogostol (Fraction A)
- 13.2 % d' -bulnesène (Fraction A)
- 9.0 % d' -guaiène (Fraction A)
- 1.8% de pachypodol co-élué à la 5-hydroxy-7,3',4'-trimethoxyflavanone (Fraction B)

### Exemple 3 : Réalisation d'un extrait de patchouli type huile essentielle

De manière conventionnelle, l'extraction de l'huile essentielle de patchouli est réalisée par distillation à la vapeur d'eau. Les parties aériennes (feuilles et tiges) de patchouli sont coupées et séchées puis sont placées dans des alambics et sont traversées par un courant de vapeur ; cette vapeur libère les molécules volatiles ou huile essentielle qui est entraînée par la vapeur d'eau et se condense dans le condenseur. Etant donné que l'huile essentielle est généralement moins dense que l'eau et n'est pas ou peu hydrosoluble, elle est recueillie à la sortie dans un décanteur appelé essencier. L'eau qui contient encore de l'huile essentielle à l'état de traces est appelée hydrolat et peut servir comme solution aromatique.

L'huile essentielle de patchouli est un liquide plus ou moins visqueux allant du jaune au brun rougeâtre. Le taux de patchoulol est compris entre 27 et 35% conformément à la norme ISO de 2003.

### Exemple 4 : Mise en évidence des différences phytochimiques majeures entre une huile essentielle et l'extrait obtenu selon l'exemple 1.

Un état de l'art antérieur poussé a été menée sur les composés constituants une huile essentielle de Patchouli. A notre connaissance, la présence de flavonoïdes ou de lipides dans ces huiles essentielles n'a jamais été répertoriée (The essential oil of patchouli, Pogostemon cablin: A review, Flavour Fragr J. 2017;1-46.)

L'extrait brut de patchouli, obtenu à l'exemple 1 a été analysé en parallèle d'une huile essentielle obtenu à l'exemple 3, par chromatographie liquide couplé à un détecteur DEDL à la même concentration. La séparation est faite sur une colonne RP-C18 en mode gradient d'élution avec des mélanges acidifiés d'eau/acétonitrile (ACN) sur la voie A et d'acétonitrile /isopropanol (IPA) sur la voie B pendant une durée de 35 minutes. Dans ces conditions, les composés phénoliques sont détectés entre 17 et 21 minutes tandis que les composés lipidiques éluent après 23 minutes. Comme le montre La figure 5, les composés phénoliques et lipidiques ne sont pas détectés dans une huile essentielle (obtenue selon l'exemple 3).

### Exemple 5 : Evaluation de l'effet de l'extrait de patchouli préparé selon l'exemple 1, sur la synthèse de collagène I dans des biopsies de peau ex vivo :

Le but de cette étude est de montrer l'effet de l'extrait de patchouli préparé selon l'exemple 1 sur la synthèse de collagène I dans des biopsies de peau saine. En effet, une réduction du taux de ce collagène est liée à l'atrophie de la matrice extracellulaire dermique, au cours du vieillissement cutané.

Protocole : Le collagène I est évalué par immuno-histochimie. Des biopsies de peau humaine saine en culture sont traitées par l'extrait de patchouli obtenu selon l'exemple 1 et formulé à 1% (masse/masse) dans une crème appliquée deux fois par jour pendant 48 heures par voie topique (20 µl/biopsie). La formulation de la crème est donnée dans le tableau 3 ci-dessous. Des biopsies contrôles reçoivent une crème placebo. Les formules utilisées sont des émulsions classiques huile-dans-eau.

Puis, la détection du collagène I est réalisée par immunomarquage à l'aide d'un anticorps spécifique.

Cette technique est réalisée à partir de coupes en paraffine, incubées en présence de l'anticorps anti-collagène I (polyclonal de lapin, Abcam). Après une heure et demi d'incubation suivie de rinçages, les coupes sont incubées en présence de l'anticorps secondaire anti-lapin couplé à un fluorophore (Alexa FluorO 488, Invitrogen). Les coupes sont ensuite examinées au microscope à Epi-fluorescence (Zeiss Axiovert 200M microscope). L'expression de collagène I est alors observée et quantifiée par analyse d'image (Volocity^{®} image analysis software, Improvision).

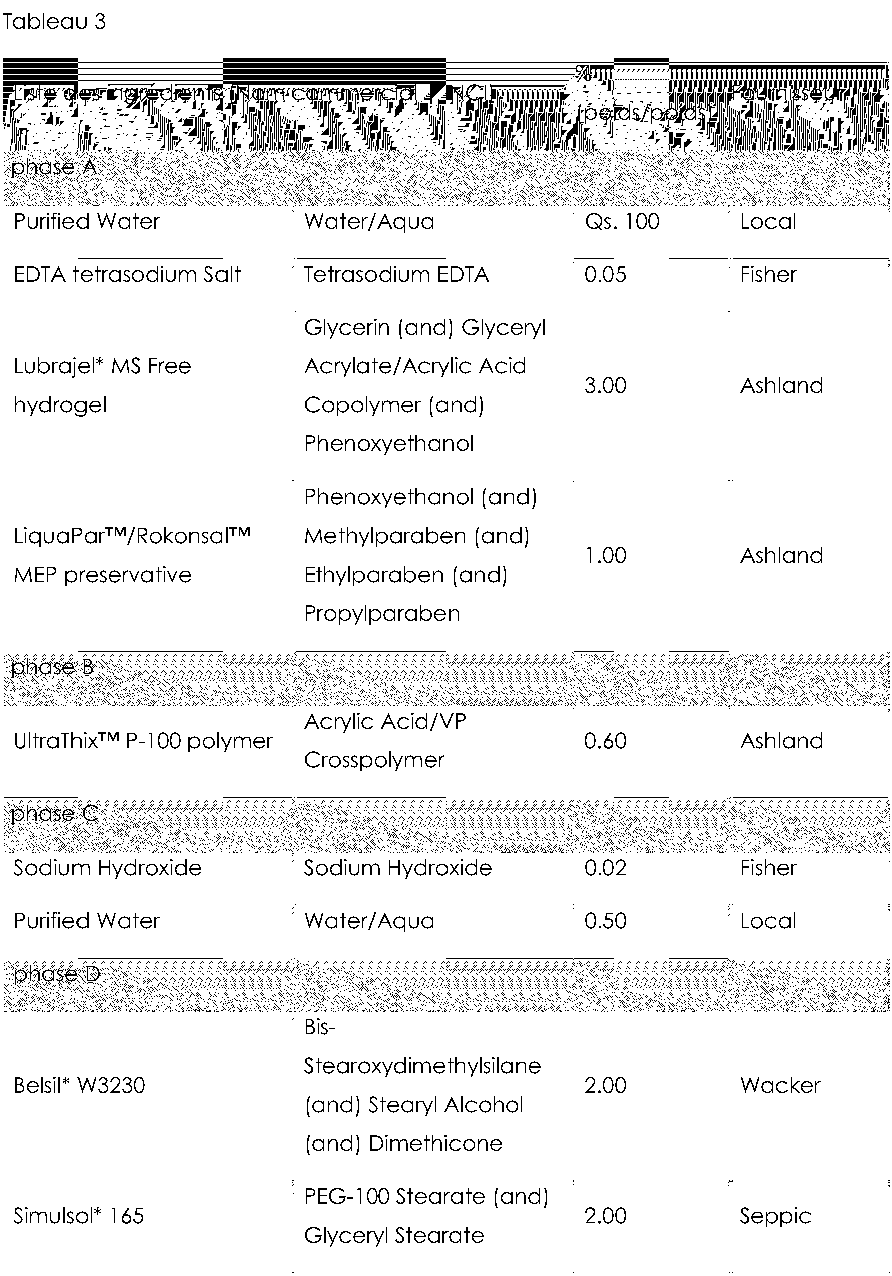

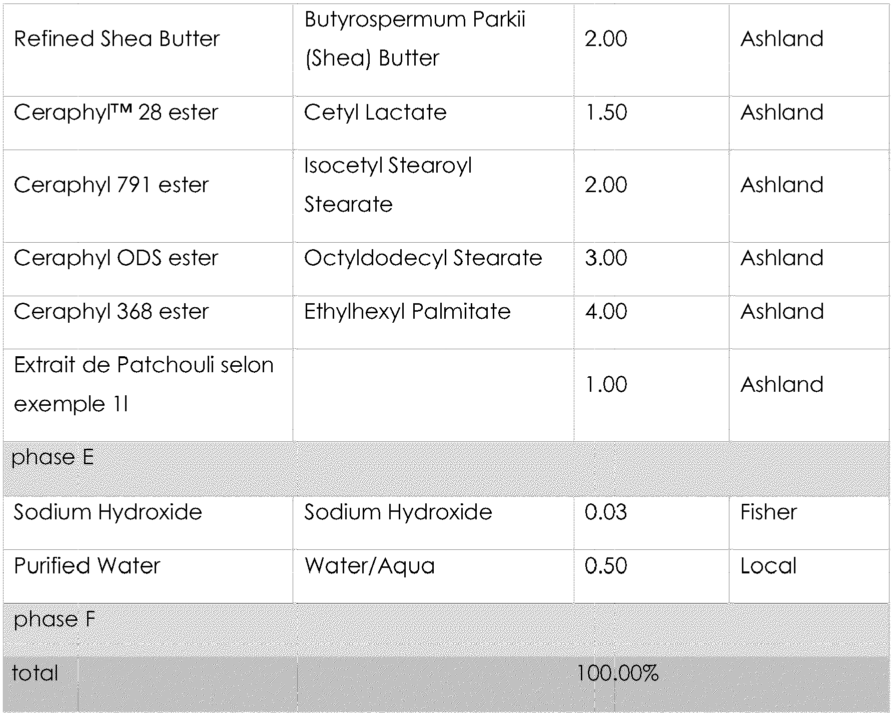

### Résultats :

Le traitement avec l'extrait de patchouli à 1% a montré une augmentation de l'intensité de marquage du collagène I dans les biopsies de peau à 48 heures (+54%, hautement significatif par rapport aux biopsies traitées avec la crème placebo).

### Conclusion :

L'extrait de patchouli à 1% a permis d'augmenter le taux de collagène I, qui est diminué au cours du vieillissement, dans des biopsies de peau saine ex vivo.

### Exemple 6 : Evaluation de l'effet de l'extrait de patchouli préparé selon l'exemple 1, sur les récepteurs cannabinoïdes CB2 dans des biopsies de peau ex vivo :

Le but de cette étude est de montrer l'effet de l'extrait de patchouli préparé selon l'exemple 1 sur les récepteurs aux cannabinoïdes CB2 dans des biopsies de peau saine.

Les récepteurs CB2 font partie du système endocannabinoïde de la peau. Des endocannabinoïdes ainsi que des cannabinoïdes exogènes peuvent agir sur ces récepteurs. Les agonistes des récepteurs CB2 sont liés à des effets anti-inflammatoires et anti-douleur.

### Protocole :

Les récepteurs CB2 sont étudiés par immuno-histochimie. Des biopsies de peau saine ont été traitées pendant 48 heures avec une crème dont la formule est donnée dans le tableau 3, contenant ou non (dans le cas du placebo) l'extrait de patchouli à 1% (20 µl par biopsie). A l'issue de ce traitement, les biopsies sont fixées par différents solvants, puis incluses dans la paraffine. Des coupes de 6 µm sont réalisées, puis incubées avec un premier anticorps spécifique des récepteurs CB2 (polyclonal de lapin, Thermofisher) pendant une heure et demi. Après des rinçages successifs, les coupes sont incubées en présence de l'anticorps secondaire anti-lapin couplé à un fluorophore (Alexa FluorO 488, Invitrogen). Les coupes sont ensuite examinées au microscope à Epi-fluorescence (Zeiss Axiovert 200M microscope). L'expression de CB2 est alors observée et quantifiée par analyse d'image (Volocity^{®} image analysis software, Improvision).

### Résultats :

Dans les biopsies de peau traitées avec l'extrait de patchouli selon l'exemple 1, on observe une augmentation de l'intensité de marquage de CB2. Cette augmentation est de +20% avec l'extrait à 0,1 % et de +36% avec l'extrait à 1%, comparé aux biopsies traitées avec la crème placebo.

### Conclusion :

L'extrait de patchouli obtenu selon l'exemple 1 a permis d'augmenter l'expression des récepteurs cannabinoïdes CB2 dans les biopsies de peau saine ex vivo. L'effet de patchouli sur les récepteurs CB2 peut être associé à une diminution de l'inflammation dans des peaux saine agressées et fragilisées.

### Exemple 7 : Evaluation de l'effet apaisant de l'extrait de patchouli à la suite d'un stress UVB :

Le but de cette étude est d'évaluer l'effet de l'extrait de patchouli obtenu selon l'exemple 1 sur le statut inflammatoire d'une peau saine soumise à une irradiation par les UVB. L'inflammation a été évaluée par caractérisation de I'IL1-R1, récepteur de l'interleukine-1, cette interleukine ainsi que son récepteur étant augmentée dans l'inflammation induite par les UVB. Une diminution de l'IL1-R1 dans les biopsies de peau exposées à un stress UVB permettra donc d'évaluer un effet apaisant de l'extrait de patchouli. Dans ce même type d'expérience, l'effet de l'extrait de patchouli obtenu selon l'exemple 1 sera comparé à celui d'une huile essentielle de patchouli à la dilution équivalente de 1%, obtenu selon l'exemple 3.

### Protocole :

Des biopsies de peau saine en culture sont exposées à une irradiation par les UVB à 200 mJ/cm2. Puis la crème contenant, ou non dans le cas du placebo, l'extrait de patchouli à 0,5 et 1%, préparé selon l'exemple 1 et formulé dans une crème selon formule donnée dans le tableau 3, est appliquée sur les biopsies (20 µL par biopsie). En parallèle, une série de biopsies est traitée avec une crème identique mais formulée avec 1% d'une huile essentielle de patchouli au lieu de l'extrait selon l'exemple 1. Après 48 heures de traitement, les biopsies sont fixées par différents solvants, puis incluses dans la paraffine. Des coupes de 6 µm sont réalisées, puis incubées avec un premier anticorps spécifique des récepteurs de l'interleukine-1 (IL1-R1 polyclonal de lapin, Tebu Rockland) pendant deux heures. Après des rinçages successifs, les coupes sont incubées en présence de l'anticorps secondaire anti-lapin couplé à un fluorophore (Alexa FluorO 488, Invitrogen). Les coupes sont ensuite examinées au microscope à Epi-fluorescence (Zeiss Axiovert 200M microscope). L'expression de l'IL1-R1 est alors observée et quantifiée par analyse d'image (Volocity^{®} image analysis software, Improvision).

### Résultats :

L'exposition des biopsies de peau ex vivo aux UVB a généré une augmentation du marquage de l'IL1-R1, révélant un état inflammatoire de la peau. Dans les biopsies irradiées et traitées avec la crème contenant l'extrait de patchouli, le taux d'IL1-R1 est plus faible, comparé au placebo. Un effet dose dépendent est observé entre 0,5% et 1% d'extrait de patchouli dans la crème (-9% et -17%, respectivement, Figure 1). La crème formulée avec l'huile essentielle de patchouli à 1% a conduit à un effet non significatif, comparé au placebo (Figure 2).

### Conclusion :

L'extrait de patchouli à 0,5 et 1% a diminué significativement le taux d'lL1-R1 dans les biopsies de peau saine irradiées avec les UVB, révélant un taux d'inflammation diminué, et par conséquent un potentiel effet apaisant de l'extrait, de manière significativement différente par rapport à l'huile essentielle de patchouli.

### Exemple 8 : Evaluation de l'effet apaisant de l'extrait de patchouli à la suite d'un stress induit par le lipopolysaccharide (LPS) bactérien :

Le but de cette expérience est de tester l'effet apaisant de l'extrait de patchouli obtenu selon l'exemple 1, sur des biopsies de peau exposé à un stress bactérien. Pour ce faire, les biopsies sont incubées en présence de lipopolysaccharide (LPS) d'origine bactérienne, composé antigénique induisant une réponse de type inflammatoire.

### Protocole :

Des biopsies de peau saine en culture sont incubées en présence de LPS à 0,5 mg/ml pendant une nuit. Puis la crème contenant, ou non dans le cas du placebo, l'extrait de patchouli à 1%, préparé selon l'exemple 1, est appliquée sur les biopsies (20 µL par biopsie). La formule de la crème est donnée dans le tableau 3. Après 48 heures de traitement, les biopsies sont fixées par différents solvants, puis incluses dans la paraffine. Des coupes de 6 µm sont réalisées, puis incubées avec un premier anticorps spécifique des récepteurs de l'interleukine-1 (IL1-R1 polyclonal de lapin, Tebu Rockland) pendant deux heures. Après des rinçages successifs, les coupes sont incubées en présence de l'anticorps secondaire anti-lapin couplé à un fluorophore (Alexa FluorO 488, Invitrogen). Les coupes sont ensuite examinées au microscope à Epi-fluorescence (Zeiss Axiovert 200M microscope). L'expression de l'IL1-R1 est alors observée et quantifiée par analyse d'image (Volocity^{®} image analysis software, Improvision).

### Résultats :

L'exposition des biopsies de peau ex vivo au LPS bactérien a généré une augmentation du marquage de l'IL1-R1, révélant un état inflammatoire de la peau. Dans les biopsies stressées par le LPS et traitées avec la crème contenant l'extrait de patchouli, le taux d'IL1-R1 est plus faible (-37%, très significatif par rapport aux biopsies stressées par le LPS et traitées avec la crème placebo) (Figure 3).

### Conclusion :

L'extrait de patchouli à 1% a diminué significativement le taux d'IL1-R1 dans les biopsies de peau saine exposées à un stress d'origine bactérienne, révélant un taux d'inflammation diminué, et par conséquent un potentiel effet apaisant de l'extrait face à une agression bactérienne.

### Exemple 9: Evaluation de l'effet de l'extrait de patchouli sur le récepteur TRPV1 à la suite d'une irradiation UVB :

Les endocannabinoïdes et les agonistes des récepteurs cannabinoïdes CB1 et CB2 peuvent exercer leur effet anti-inflammatoire en agissant sur d'autres récepteurs tels que TRPV1 (Transient Receptor Potential Vanilloide 1) associé à la nociception et au prurit (Michael J. Caterina. TRP Channel Cannabinoid Receptors in Skin Sensation, Homeostasis, and Inflammation. ACS Chem. Neurosci. 2014, 5, 1107-1116).

Le récepteur TRPV1 (Transient Receptor Potential Vanilloide 1) agit comme un détecteur de différents stress associés à une perception douloureuse (nociception), incluant la chaleur, une baisse de pH, ainsi que l'activation par des médiateurs pro inflammatoires endogènes. Son activation se traduit par une réaction inflammatoire, pouvant s'accompagner d'une perception d'inconfort, de démangeaisons, comme dans le cas du prurit, voire même d'une sensation douloureuse.

TRPV1 est un récepteur pour les endocannabinoïdes et cannabinoïdes exogènes, qui peuvent exercer un effet analgésique (Michael J. Caterina. TRP Channel Cannabinoid Receptors in Skin Sensation, Homeostasis, and Inflammation. ACS Chem. Neurosci. 2014, 5, 1107-1116*).*

Dans la présente étude, l'effet de l'extrait de patchouli obtenu selon l'exemple 1 sur les récepteurs TRPV1 a été évalué sur des biopsies de peau en culture, exposées à un stress UVB.

### Protocole :

Des biopsies de peau saine en culture sont exposées à une irradiation par les UVB à 200 mJ/cm2. Puis la crème contenant, ou non dans le cas du placebo, l'extrait de patchouli à 1%, préparé selon l'exemple 1, est appliquée sur les biopsies (20 µL par biopsie). La formule de la crème est donnée dans le tableau 3. Après 48 heures de traitement, les biopsies sont fixées par différents solvants, puis incluses dans la paraffine. Des coupes de 6 µm sont réalisées, puis incubées avec un premier anticorps spécifique des récepteurs TRPV1 (polyclonal de lapin, Invitrogen) pendant deux heures. Après des rinçages successifs, les coupes sont incubées en présence de l'anticorps secondaire anti-lapin couplé à un fluorophore (Alexa FluorO 488, Invitrogen). Les coupes sont ensuite examinées au microscope à Epi-fluorescence (Zeiss Axiovert 200M microscope). L'expression de TRPV1 est alors observée et quantifiée par analyse d'image (Volocity^{®} image analysis software, Improvision).

### Résultats :

Les biopsies de peau exposées à un stress UVB ont montré une augmentation du marquage de TRPV1 qui, selon la bibliographie, est associé à une augmentation de l'état inflammatoire. Dans les biopsies de peau traitées avec l'extrait de patchouli obtenu selon l'exemple 1, on observe une baisse de l'intensité de marquage de TRPV1, indiquant une réduction de l'état inflammatoire induit par les UVB (-31%, hautement significatif par rapport aux biopsies irradiées aux UVB et traitées par la crème placebo) (Figure4).

### Conclusion :

Le traitement des biopsies de peau saine par l'extrait de patchouli a montré une réduction de l'expression de TRPV1 dans la peau saine irradiée par les UVB, associée à une baisse de l'inflammation et de la sensibilité cutanée.

### Exemple 10 : Evaluation de l'effet de l'extrait de patchouli préparé selon l'exemple 1, sur la synthèse de loricrine dans des biopsies de peau ex vivo :

Le but de cette étude est de montrer l'effet de l'extrait de patchouli préparé selon l'exemple 1 sur la synthèse de loricrine dans des biopsies de peau ex vivo. En effet, cette protéine est impliquée dans le processus de différenciation de l'épiderme et participe donc à l'élaboration de la barrière cutanée, en lien avec l'hydratation de la peau.

### Protocole :

La loricrine est évaluée par immuno-histochimie. Des biopsies de peau saine humaine en culture sont traitées par l'extrait de patchouli obtenu selon l'exemple 1 et formulé à 0.5% (masse/masse) dans une crème appliquée deux fois par jour pendant 48 heures par voie topique (20 µl/biopsie). La formule de la crème est donnée dans le tableau 3. Des biopsies contrôles reçoivent une crème placebo. Les formules utilisées sont des émulsions classiques huile-dans-eau.

Puis, la détection de la loricrine est réalisée par immunomarquage à l'aide d'un anticorps spécifique.

Cette technique est réalisée à partir de coupes en paraffine, incubées en présence de l'anticorps anti-loricrine (polyclonal de lapin, Abcam). Après une heure et demi d'incubation suivie de rinçages, les coupes sont incubées en présence de l'anticorps secondaire anti-lapin couplé à un fluorophore (Alexa FluorO 488, Invitrogen). Les coupes sont ensuite examinées au microscope à Epi-fluorescence (Zeiss Axiovert 200M microscope). L'expression de loricrine est alors observée et quantifiée par analyse d'image (Volocity^{®} image analysis software, Improvision).

### Résultats :

Le traitement avec l'extrait de patchouli à 0.5% a montré une augmentation de l'intensité de marquage de la loricrine dans les biopsies de peau à 48 heures (+39%, hautement significatif par rapport aux biopsies traitées avec la crème placebo).

### Conclusion :

L'extrait de patchouli a permis d'augmenter le taux de loricrine dans les biopsies de peau saine ex vivo, en relation avec un effet sur la différenciation de l'épiderme et la fonction barrière cutanée.

## Revendications

1. Procédé d'obtention d'un extrait de feuilles de patchouli comprenant les étapes suivantes :
a. La partie haute des parties aériennes de patchouli est récoltée puis séchée et broyée ou cryobroyée.
b. on réalise une extraction au dioxyde de carbone supercritique en présence d'éthanol à une concentration comprise entre 60 et 80% (pourcentage volume/volume), en tant que co-solvant polaire.
c. on récupère l'extrait brut de patchouli qui est décoloré en présence de charbon actif et filtré au préalable de l'évaporation du co-solvant.

2. Procédé selon la revendication 1 dans lequel à l'étape b) le co-solvant est de l'éthanol à une concentration de 70% (pourcentage volume/volume).

3. Procédé selon l'une des revendications 1 ou 2 dans lequel à l'étape b) le ratio massique entre le dioxyde de carbone supercritique et le co-solvant est de 15 et le rapport massique entre le dioxyde de carbone supercritique et la quantité de matière première mise en jeu est compris entre 10 et 70, avantageusement entre 25 et 55 et préférentiellement entre 30 et 50.

4. Procédé selon l'une des revendications 1 à 3, dans lequel à l'étape b) la température d'extraction est comprise entre 35 et 70°C, avantageusement entre 40 et 65°C et préférentiellement entre 50 et 60°C et la pression sein de l'extracteur est comprise entre 90 et 1000 bar, préférentiellement entre 160 et 800 bar et encore plus préférentiellement entre 230 et 700 bar.

5. Procédé selon l'une des revendications 1 à 4 dans lequel l'extrait brut obtenu à l'étape c) est solubilisé dans un solvant de type alcool gras saturé ou insaturé, linéaire ou ramifié, comprenant de 8 à 30 carbones pour obtenir une concentration d'extrait brut dans l'extrait final solubilisé comprise entre 2 et 6 % en poids du poids total de l'extrait solubilisé.

6. Extrait brut de de feuilles de patchouli susceptible d'être obtenu par le procédé de l'une des revendications 1 à 4, **caractérisé en ce qu'**il comprend de 50 à 80% de composés volatils (principalement des sesquiterpènes et des alcools sesquiterpéniques), avantageusement de 55 à 75% et préférentiellement de 60 à 70%; de 15 à 48% de composés lipidiques (notamment des acides gras, phytostérols, triterpènes, acyl glycérides), avantageusement 20.5 à 42.5% et préférentiellement de 26 à 32% et enfin, de 2 à 5% de composés phénoliques (principalement des flavonoïdes), avantageusement 2.5 à 4.5% et préférentiellement de 3 à 4% .

7. Composition cosmétique pour le soin de la peau et des phanères, comprenant comme agent actif, un extrait solubilisé de patchouli obtenu selon le procédé de la revendication 5 et un milieu physiologiquement acceptable.

8. Composition cosmétique selon la revendication 7, dans laquelle l'extrait solubilisé de patchouli est à une concentration comprise entre 0,1 et 10 %, préférentiellement entre 0,5 et 5 %.

9. Utilisation cosmétique d'une composition selon la revendication 7 pour le soin de la peau, du cuir chevelu et des phanères.

10. Utilisation cosmétique selon la revendication 9 pour améliorer l'aspect de la peau, lutter contre les signes du vieillissement cutané ou pour améliorer l'hydratation de la peau et renforcer la fonction barrière.

11. Utilisation cosmétique selon la revendication 9 pour apaiser la peau.

12. Utilisation cosmétique selon la revendication 9 pour augmenter l'expression des récepteurs endocannabinoïdes CB2 dans la peau.

13. Utilisation cosmétique selon la revendication 9 pour diminuer l'expression des récepteurs endocannabinoïdes TRPV1 dans la peau, après irradiation par les UVB.

## Patentansprüche

1. Verfahren zum Erhalten eines Extrakts aus Blättern von Patschuli, umfassend die folgenden Schritte:
a. der obere Teil der oberirdischen Teile von Patschuli wird geerntet, dann getrocknet und zerkleinert oder kryogen zerkleinert,
b. eine Extraktion mit superkritischem Kohlendioxid in Anwesenheit von Ethanol bei einer Konzentration zwischen 60 und 80 % (Prozentsatz Volumen/Volumen) als polares Co-Lösungsmittel wird durchgeführt,
c. der Rohextrakt aus Patschuli wird in Anwesenheit von Aktivkohle entfärbt und vor dem Abdampfen des Co-Lösungsmittels filtriert.

2. Verfahren nach Anspruch 1, wobei in dem Schritt b) das Co-Lösungsmittel Ethanol bei einer Konzentration von 70 % (Prozentsatz Volumen/Volumen) ist.

3. Verfahren nach einem der Ansprüche 1 oder 2, wobei in dem Schritt b) das Masseverhältnis zwischen dem superkritischen Kohlendioxid und dem Co-Lösungsmittel 15 beträgt, und das Masseverhältnis zwischen dem superkritischen Kohlendioxid und der Menge an verwendetem Ausgangsmaterial zwischen 10 und 70 beträgt, vorteilhaft zwischen 25 und 55 und bevorzugt zwischen 30 und 50.

4. Verfahren nach einem der Ansprüche 1 bis 3, wobei in dem Schritt b) die Extraktionstemperatur zwischen 35 und 70 °C beträgt, vorteilhaft zwischen 40 und 65 °C und bevorzugt zwischen 50 und 60 °C, und der Druck im Inneren des Extraktors zwischen 90 und 1000 bar beträgt, bevorzugt zwischen 160 und 800 bar und noch bevorzugter zwischen 230 und 700 bar.

5. Verfahren nach einem der Ansprüche 1 bis 4, wobei der Rohextrakt, der in dem Schritt c) erhalten wird, in einem Lösungsmittel vom Typ eines gesättigten oder ungesättigten, geraden oder verzweigten Fettalkohols mit 8 bis 30 Kohlenstoffatomen solubilisiert wird, um eine Konzentration des Rohextrakts in dem solubilisierten Endextrakt zwischen 2 und 6 Gew.-%, bezogen auf das Gesamtgewicht des solubilisierten Extrakts, zu erhalten.

6. Rohextrakt aus Blättern von Patschuli, welcher durch das Verfahren nach einem der Ansprüche 1 bis 4 erhalten werden kann, **dadurch gekennzeichnet, dass** er von 50 bis 80 % flüchtige Verbindungen umfasst (hauptsächlich Sesquiterpene und Sesquiterpenalkohle), vorteilhaft von 55 bis 75 % und bevorzugt von 60 bis 70 %; von 15 bis 48 % Lipidverbindungen (insbesondere Fettsäuren, Phytosterole, Triterpene, Acylglyceride), vorteilhaft von 20,5 bis 42,5 % und bevorzugt von 26 bis 32 %, und schließlich von 2 bis 5 % Phenolverbindungen (hauptsächlich Flavonoide), vorteilhaft 2,5 bis 4,5 % und bevorzugt von 3 bis 4 %.

7. Kosmetische Zusammensetzung zur Pflege der Haut und der Hautanhangsgebilde, umfassend, als aktives Mittel, einen solubilisierten Extrakt aus Patschuli, der nach dem Verfahren von Anspruch 5 erhalten wird, und ein physiologisch annehmbares Medium.

8. Kosmetische Zusammensetzung nach Anspruch 7, wobei der solubilisierte Extrakt aus Patschuli in einer Konzentration zwischen 0,1 und 10 % vorhanden ist, bevorzugt zwischen 0,5 und 5 %.

9. Kosmetische Verwendung einer Zusammensetzung nach Anspruch 7 zur Pflege der Haut, der Kopfhaut und der Hautanhangsgebilde.

10. Kosmetische Verwendung nach Anspruch 9 zur Verbesserung des Aussehens der Haut, zur Bekämpfung der Zeichen der Hautalterung oder zur Verbesserung der der Feuchtigkeit der Haut und Verstärkung ihrer Barrierefunktion.

11. Kosmetische Verwendung nach Anspruch 9 zur Beruhigung der Haut.

12. Kosmetische Verwendung nach Anspruch 9 zur Erhöhung der Expression der Endocannabinoid-Rezeptoren CB2 in der Haut.

13. Kosmetische Verwendung nach Anspruch 9 zur Verringerung der Expression der Endocannabinoid-Rezeptoren TRPV1 in der Haut nach Bestrahlung mit UVB.

## Claims

1. A method for obtaining an extract of patchouli leaves comprising the following steps:
a. harvesting the upper part of the patchouli aerial parts and then drying and grounding or cryogenically grounding.
b. performing a supercritical carbon dioxide extraction in the presence of ethanol at a concentration of between 60 and 80% (volume/volume) as a polar co-solvent.
c. recovering the crude patchouli extract, decolouring it in the presence of activated carbon and filtering before the co-solvent is evaporated.

2. The method according to claim 1, wherein in step b), the co-solvent is ethanol at a concentration of 70% (volume/volume percentage).

3. The method according to claim 1 or 2, wherein in step b) the weight ratio between the supercritical carbon dioxide and the co-solvent is 15 and the weight ratio between the quantity of raw material used is comprised between 10 and 70, advantageously between 25 and 55 and preferably between 30 and 50.

4. The method according to claim 1 to 3, wherein in step b) the extraction temperature is comprised between 35 and 70° C, advantageously between 40 and 65° C and preferably between 50 and 60° C, and the pressure within the extractor is comprised between 90 and 1000 bar, preferably between 160 and 800 bar and even more preferably between 230 and 700 bar.

5. The method according to claim 1 to 4, wherein the crude extract obtained in step c) is solubilized in a solvent such as a saturated or unsaturated, linear or branched fatty alcohol comprising 8 to 30 carbon atoms to obtain a crude extract concentration in the final solubilised extract comprised between 2 and 6% by weight of the total weight of the solubilized extract.

6. A crude extract of patchouli leaves obtained by the method according to claim 1 to 4, **characterized in that** it comprises from 50 to 80% of volatile compounds (mainly of sesquiterpenes and sesquiterpene alcohols), advantageously from 55 to 75% and preferably from 60 to 70%; from 15 to 48% of lipid compounds (in particular fatty acids, phytosterols, triterpenes, acyl glycerides), advantageously from 20.5 to 42.5% and preferably from 26 to 32%, and lastly from 2 to 5% of phenolic compounds (primarily flavonoids), advantageously 2.5 to 4.5% and preferably from 3 to 4%.

7. A cosmetic composition for the care of the skin and the appendages, comprising as an active ingredient, a solubilised patchouli extract obtained by the method according to claim 5, and a physiologically acceptable medium.

8. The cosmetic composition according to claim 7, in which the solubilised patchouli extract is at a concentration comprised between 0.1 and 10%, preferably between 0.5 and 5%.

9. Cosmetic use of a composition according to claim 7 for the care of the skin, the scalp and the appendages.

10. Cosmetic use according to claim 9 to improve the appearance of the skin, to combat the signs of skin ageing or to improve the hydration of the skin and reinforce the barrier function.

11. Cosmetic use according to claim 9 for soothing the skin.

12. Cosmetic use according to claim 9 for increasing the expression of CB2 endocannabinoid receptors in the skin.

13. Cosmetic use according to claim 9 for decreasing the expression of TRPV1 endocannabinoid receptors in the skin, after UVB irradiation.
